(19) Europäisches Patentamt
European Patent Office
Office européen des brevets

(11) Veröffentlichungsnummer: **0 248 423 B1**

# (12) EUROPÄISCHE PATENTSCHRIFT

(45) Veröffentlichungstag der Patentschrift: **30.08.89**

(51) Int. Cl.⁴: **C07C 45/46**, C07C 49/76, C07C 65/34, C07C 51/373

(21) Anmeldenummer: **87108066.9**

(22) Anmeldetag: **04.06.87**

## (54) Verfahren zur Acylierung von Aromaten.

(30) Priorität: **06.06.86 DE 3619169**
**11.12.86 DE 3642329**

(43) Veröffentlichungstag der Anmeldung:
**09.12.87 Patentblatt 87/50**

(45) Bekanntmachung des Hinweises auf die Patenterteilung:
**30.08.89 Patentblatt 89/35**

(84) Benannte Vertragsstaaten:
**AT BE CH DE ES FR GB IT LI NL SE**

(56) Entgegenhaltungen:
**EP-A- 0 012 850**
**DE-A- 2 720 294**
**DE-B- 1 288 584**
**US-A- 4 310 705**

(73) Patentinhaber: **BASF Aktiengesellschaft,
Carl-Bosch-Strasse 38, D-6700 Ludwigshafen(DE)**

(72) Erfinder: **Eggersdorfer, Manfred, Dr.,
Hannongstrasse 18, D-6710 Frankenthal(DE)**
Erfinder: **Siegel, Hardo, Dr., Hans-Purrmann-Allee 25,
D-6720 Speyer(DE)**
Erfinder: **Schuhmacher, Alfred, Dr.,
Von-Weber-Strasse 36, D-6700 Ludwigshafen(DE)**
Erfinder: **Grosch, Walter, Dr., Starenweg 1,
D-6803 Edingen-Neckarhausen(DE)**
Erfinder: **Henkelmann, Jochem, Dr., Ludwigshafener
Strasse 44, D-6704 Mutterstadt(DE)**

EP 0 248 423 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein verbessertes Verfahren zur Acylierung von Aromaten nach Friedel-Crafts, insbesondere zur Herstellung von alkylsubstituierten aromatischen Ketonen durch Umsetzung von Carbonsäurehalogeniden oder Carbonsäureanhydriden mit Alkylaromaten in Gegenwart eines Friedel-Crafts-Katalysators.

Die Friedel-Craft-Acylierung von Aromaten, d.h. die Einführung einer Acylgruppe in aromatische Verbindungen durch Einwirkung eines Acylierungsmittels auf Aromaten in Gegenwart bestimmter Metallhalogenide wie beispielsweise Aluminiumchlorid, ist allgemein bekannt, z.B. aus Houben-Weyl, Methoden der org. Chem. Bd. VII/2a, 1973, Seiten 15-39 und 311-325. Nachteilig an diesem Verfahren ist das Auftreten von Nebenreaktionen, insbesondere wenn alkylsubstituierte Aromaten acyliert werden. So kommt es zur Bildung harziger Nebenprodukte, sowie zur Abspaltung der Alkylgruppe und zu Isomerisierungen, vor allem wenn Aromaten mit sekundären oder tertiären Alkylresten umgesetzt werden. Wie aus verschiedenen Veröffentlichungen hervorgeht, sollen diese Nebenreaktionen durch ein Zusammenwirken von $AlCl_3$ und Chlorwasserstoff, der, sofern er nicht im Katalysator gegenwärtig ist, während der Friedel-Crafts-Acylierung entsteht (s. C.A. Olah, Friedel-Crafts and Related Reactions, Bd. I, Seite 207 und Bd. III, Teil I, S. 550 ff, Intersience 1964), verursacht werden. Um die Nebenreaktionen zurückzudrängen, wird empfohlen, frisch sublimierte Katalysatoren zu verwenden und den entstehenden Halogenwasserstoff durch Anlegen von vermindertem Druck oder Durchleiten von Inertgasen aus dem Reaktionsgemisch zu entfernen (s. C.A. Olah, Friedel-Crafts and Related Reactions, Bd. III, S. 549 und dort zitierte Literatur). Durch diese Maßnahmen kann, wie auch aus der DE-AS 27 20 294 hervorgeht, die Isomerisierung in gewissem Maße zurückgedrängt werden, jedoch ist das Ergebnis noch unbefriedigend; die weitere Auftrennung der anfallenden Produktgemische ist aufwendig und in vielen Fällen nicht wirtschaftlich möglich, so daß die entsprechenden Gemische für Folgeumsetzungen eingesetzt werden müssen. Weiterhin ist nachteilig, daß erhebliche Mengen der Reaktanten aus dem Reaktionsgemisch entfernt werden, wodurch schlechte Ausbeuten resultieren.

Der Erfindung lag daher die Aufgabe zugrunde, die Friedel-Crafts-Acylierung von Aromaten so zu verbessern, daß Nebenreaktionen weitgehend vermieden werden und die Produkte in hohen Ausbeuten anfallen. Insbesondere sollte bei der Umsetzung von Alkylaromaten die Abspaltung und Isomerisierung der Alkylgruppen unterdrückt werden.

Es wurde nun ein Verfahren zur Acylierung von Aromaten nach Friedel-Crafts gefunden, das dadurch gekennzeichnet ist, daß man die Acylierung in Gegenwart von Metallalkylen oder Metallalkylhalogeniden von Metallen oder Halbmetallen der zweiten bis fünften Hauptgruppe und/oder Metallen der zweiten oder vierten Nebengruppe des Periodensystems durchführt.

Besonders vorteilhaft können nach dem erfindungsgemäßen Verfahren Carbonsäurehalogenide oder -anhydride mit Alkylaromaten zu alkylsubstituierten Ketonen umgesetzt werden. Bei Verwendung von a) Acetylchlorid bzw. b) Phthalsäureanhydrid und tert.-Butylbenzol als Ausgangsstoffe und einem Gemisch aus Metallalkyl bzw. Metallalkylhalogenid und Aluminiumchlorid als Katalysator läßt sich die Umsetzung durch folgende Reaktionsgleichungen beschreiben:

a)

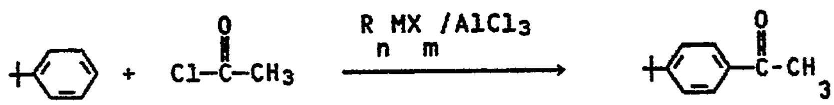


b)

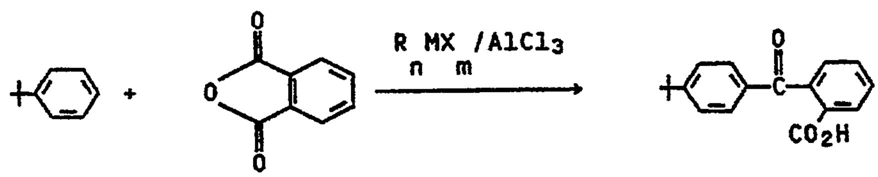


R= Alkylrest
X= Halogen
M= Metall bzw. Halbmetall
n= 1 bis 4
m= 0 bis 4
wobei m + n = Wertigkeit des Metalls

Erfindungsgemäß wird die Friedel-Crafts Acylierung in Gegenwart wirksamer Mengen an Metallalkylen bzw. Metallalkylhalogeniden von Metallen oder Halbmetallen der zweiten bis fünften, insbesondere der dritten Hauptgruppe des Periodensystems sowie Metallen der zweiten und/oder vierten Nebengruppe durchgeführt. Sie lassen sich durch die allgemeine Formel

$R_nMX_m$ I

wiedergeben, worin der Rest R gleiche oder verschiedene Alkylgruppen mit jeweils 1 bis 12, vorzugswei-

se 1 bis 6, insbesondere 1 bis 4 Kohlenstoffatomen z.B. Methyl-, Ethyl-, n-Propyl-, iso-Propyl-, n-Butyl- oder iso-Butylgruppen bedeutet, M Metalle bzw. Halbmetalle der genannten Gruppen, z.B. Beryllium, Magnesium, Aluminium, Bor, Gallium, Indium, Thallium, Silicium, Zinn, Blei, Antimon, Zink, Cadmium, Quecksilber und Titan darstellt, X für Fluor, Chlor, Brom oder Jod steht, wobei aus Kostengründen häufig Chlor bevorzugt ist, n die Zahlen 1 bis 4 und m die Zahlen 0 bis 4 bedeuten, wobei die Summe n plus m die Wertigkeit des Metalls ergibt.

Auch Gemische verschiedener Verbindungen I sind möglich.

Die Herstellung der metallorganischen Verbindungen I kann in an sich bekannter Weise, z.B. wie in Brockhaus ABC Chemie, Bd. 2, S. 867-869, 1971 beschrieben, erfolgen.

Für das erfindungsgemäße Verfahren besonders geeignet sind metallorganische Verbindungen von Magnesium wie Dialkylmagnesium oder Grignardverbindungen (RMgX), Bor wie Trialkylbor, Dialkylborhalogenid oder Alkylbordihalogenid, Zinn wie Tetraalkylzinn oder Trialkylzinnhalogenide, Titan wie Alkyltitantrihalogenid oder Dialkyltitandihalogenid sowie zink-und cadmiumorganische Verbindungen wie Dialkylzink oder Dialkylcadmium. Bevorzugt können insbesondere zinkorganische Verbindungen verwendet werden. Besonders bevorzugt sind Aluminiumalkyle bzw. Alkylaluminiumhalogenide z.B. solche der Formel I a

$R_nAlX_{3-n}$I a

in der R gleiche oder verschiedene Alkylgruppen mit jeweils 1 bis 12, vorzugsweise 1 bis 6, insbesondere 1 bis 4 Kohlenstoffatomen z.B. Methyl-, Ethyl-, n-Propyl-, iso-Propyl-, n-Butyl- oder iso-Butylgruppen bedeutet, X für Fluor, Chlor, Brom oder Jod steht, wobei aus Kostengründen Chlor bevorzugt ist, n die Zahlen 1, 2 oder 3 darstellt oder Gemische von zwei verschiedenen Verbindungen der Formel Ia.

Beispielsweise seien folgende Verbindungen aufgeführt:
Methylmagnesiumchlorid, Ethylmagnesiumbromid, Diethylmagnesium, Trimethylbor, Triethylbor, Diethylborchlorid, Diethylborbromid, Ethylbordichlorid, Tetramethylzinn, Triethylzinnchlorid, Methyltitantrichlorid, Diethyltitandichlorid, Dimethylcadmium und insbesondere Dimethylzink, Diethylzink, Dipropylzink sowie Dibutylzink und Ethylzinkchlorid.

Besonders bevorzugte Verbindungen sind: Methylaluminiumdichlorid und -dibromid, Ethylaluminiumdichlorid und -dibromid, iso-Propylaluminiumdibromid, n-Hexylaluminiumdichlorid, Dodecylaluminiumdijodid, Dimethylaluminiumchlorid, Diethylaluminiumbromid oder Dibutylaluminiumiodid. Gemische dieser Verbindung sind beispielsweise Methylaluminiumsesquichlorid, Ethylaluminiumsesquichlorid oder Gemische von Alkylaluminiumdichlorid und Dialkylaluminiumchlorid in einem Molverhältnis von z.B. 20 : 1 bis 1 : 20.

Anstelle der Alkylaluminiumhalogenide kann dem Reaktionsgemisch auch vorteilhaft Trialkylaluminium zugesetzt werden, z.B. Trimethyl-, Triethyl-, Tri-n-propyl-, Triisopropyl-, Tri-n-butyl- oder Tri-n-hexylaluminium sowie Aluminiumalkyle mit gemischten Alkylresten, z.B. Methyldiethylaluminium.

Als Friedel-Crafts-Katalysatoren können die an sich üblichen Verbindungen wie $FeCl_3$, $BF_3$, $ZnCl_2$ oder $TiCl_4$ verwendet werden. Besonders geeignet sind Aluminiumhalogenide, bevorzugt Aluminiumbromid und insbesondere Aluminiumchlorid.

Als Aromaten kommen die üblicherweise für Friedel-Crafts-Reaktionen verwendeten Verbindungen wie isocyclische und heterocyclische aromatische Kohlenwasserstoffe in Betracht. Nach dem erfindungsgemäßen Verfahren lassen sich besonders vorteilhaft Alkylaromaten, z.B. alkylsubstituierte Benzole, Naphthaline, Anthracene, Furane, Benzofurane, Thiophene usw. umsetzen. Alkylreste sind z.B. solche mit 1 bis 20, insbesondere 1 bis 12, vorzugsweise 1 bis 8 Kohlenstoffatomen. Der Alkylrest sowie der aromatische Kern können noch weitere Substituenten wie Halogen, z.B. Chlor oder Brom oder $C_1$-$C_4$-Alkyl- oder -Alkoxyreste oder Hydroxygruppen tragen, ferner können im Alkylrest Doppel- oder Dreifachbindungen enthalten sein. Vorzugsweise werden Alkylbenzole mit 1 oder 2 verzweigten oder unverzweigten Alkylresten umgesetzt. Beispielsweise seien folgende Alkylbenzole genannt:

Toluol, Ethylbenzol, n-Propylbenzol, iso-Propylbenzol, n-Butylbenzol, sek.-Butylbenzol, tert.-Butylbenzol, n-Pentylbenzol, (2-Methylbutyl)-benzol, (3-Methylbutyl)benzol, (1-Methylbutyl)-benzol, (1,1-Dimethylpropyl)benzol, n-Hexylbenzol, (1-Ethyl-1-methylpropyl)benzol, (1,1-Dimethylbutyl)benzol, (1-Methylpentyl)-benzol, (1-Ethyl-1-methyl)-benzol, (1-Ethylhexyl)benzol, (4-Octyl)-benzol, 1,2-Dimethylbenzol oder 1,4-Diethylbenzol.

Als Acylierungsmittel dienen die üblicherweise verwendeten Verbindungen, insbesondere Carbonsäurehalogenide und -anhydride, aber auch -imidchloride oder die Carbonsäuren selbst.

Hinsichtlich der umzusetzenden Carbonsäurehalogenide bzw. Carbonsäureanhydride bestehen keine Beschränkungen, so lassen sich die Säurefluoride, -iodide und insbesondere -bromide und -chloride umsetzen. In der Regel wird man die Säurechloride aliphatischer , cycloaliphatischer, araliphatischer, aromatischer und heterocyclischer Carbonsäuren umsetzen. Beispielsweise seien Säurechloride der Formel $R^1COCl$ aufgeführt, in der $R^1$ Wasserstoff oder einen aliphatischen Rest darstellt, z.B. einen Alkylrest mit 1 bis 20, insbesondere 1 bis 8 Kohlenstoffatomen, einen gegebenenfalls substituierten Arylrest, vorzugsweise einen Phenylrest, der, wenn er substituiert ist, insbesondere 1 bis 2 Substituenten wie $C_1$- bis $C_4$-Alkylreste, Aryloxy- wie Phenoxy-, Halogen wie Fluor, Chlor oder Brom oder Nitrogruppen tragen kann. Darüber hinaus kann $R^1$ einen Aralkylrest wie Benzylrest oder einen heterocyclischen Rest, vorzugsweise einen Sauerstoff oder Schwefel enthaltenden heterocyclischen Rest mit 5 oder 6 Ringgliedern, z.B. einen Furanyl-, Pyranyl- oder Thiophenrest bedeuten.

Beispielsweise seien folgende Säurechloride genannt:

Formylchlorid, Acetylchlorid, Propionylchlorid, n-Butyrylchlorid, n-Octadecansäurechlorid, 3,3-Dimethylacrylsäurechlorid, Benzoylchlorid, 3-Methoxybenzoylchlorid, 3-Phenoxybenzoylchlorid, o-Chlorbenzoylchlorid, 3-Chlor-5-methylbenzoylchlorid, 2,6-Dichlorbenzoylchlorid, m-Brombenzoylchlorid, o-Brombenzoylchlorid, p-Methylbenzoylchlorid, p-tert.-Butyl-benzoylchlorid, p-Nitrobenzoylchlorid, p-Carbomethoxybenzoylchlorid, m-Carbobutoxybenzoylchlorid, Phenylessigsäurechlorid, 4-Chlorphenylessigsäurechlorid, Zimtsäurechlorid, 4-Chlorzimtsäurechlorid, Furan-2-carbonsäurechlorid, Thiophen-2-carbonsäurechlorid.

Als Säureanhydride können Anhydride von aliphatischen, cycloaliphatischen, araliphatischen, aromatischen oder heterocyclischen Mono- oder Dicarbonsäuren eingesetzt werden. Anhydride von Monocarbonsäuren sind beispielsweise solche von Alkylcarbonsäuren wie Acetanhydrid, Propionsäureanhydrid, n-Buttersäureanhydrid oder Benzoesäureanhydrid, Anhydride von Dicarbonsäuren sind beispielsweise solche der Formel

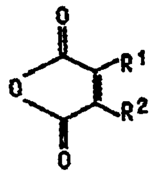

in der $R^2$ die Bedeutung von $R^1$ hat und darüberhinaus $R^1$ und $R^2$ miteinander ringförmig zu einem gegebenenfalls substituierten aromatischen System, z.B. einen Benzolring verbunden sein können. Beispielsweise seien Maleinsäureanhydrid, Bernsteinsäureanhydrid, Methylmaleinsäureanhydrid, Tetraethylbernsteinsäureanhydrid, Phthalsäureanhydrid oder 4,5-Methylendioxy-phthalsäureanhydrid genannt.

Zweckmäßigerweise können stöchiometrische Mengen an aromatischer Verbindung und Acylierungsmittel verwendet werden. Es ist aber auch möglich, eine der beiden Komponenten gegenüber der anderen im Überschuß einzusetzen, so kann man beispielsweise 1 bis 1,5 mol Aromat je Mol Acylierungsmittel verwenden.

Die für Friedel-Crafts-Reaktionen üblicherweise verwendeten Katalysatormengen können auch im erfindungsgemäßen Verfahren eingesetzt werden, wobei sich die Gesamtkatalysatormenge aus der Summe der Lewis-Säure wie Aluminiumhalogenid und der metallorganischen Verbindung I ergibt.

Der Zusatz der metallorganischen Verbindung bewirkt, daß der im Reaktionsgemisch vorhandene bzw. entstehende Chlorwasserstoff gebunden wird. Die wirksame Menge richtet sich daher nach der Anzahl der im Molekül vorhandenen Alkylreste. Durch den Anteil an Metallalkyl oder Metallalkylhalogenid kann das Verhältnis der Isomerisierungsprodukte (z.B. im Falle tert.-amylsubstituierter Aromaten, das tert./sek.-Alkylverhältnis) und weiterhin die Menge an Nebenprodukten beeinflußt werden. In der Regel können pro Äquivalent Carbonsäurehalogenid 0,1 bis etwa 1,1 Metallalkyläquivalente als Metallalkyl oder Metallalkylhalogenid und 0,1 bis 1,5 mol Friedel-Crafts-Katalysatoren, z.B. Aluminiumhalogenid zugesetzt werden, wobei es vorteilhaft ist, einen geringen, z.B. 10 %igen Überschuß des Friedel-Crafts-Katalysators, zu verwenden. Höhere Überschüsse können eingesetzt werden, bringen aber keinen Vorteil. Bei Carbonsäureanhydriden oder Carbonsäurehalogniden, die Substituenten tragen, welche mit Friedel-Crafts-Katalysatoren, z.B. Aluminiumhalogeniden und metallorganischen Verbindungen stabile Komplexe bilden, werden pro Substituent ein Äquivalent an Katalysator zusätzlich benötigt.

Gemäß der bevorzugten Ausführungsform des Verfahrens können pro Äquivalent Säurehalogenid 1 bis 1,5 mol, vorzugsweise 1,1 bis 1,2 mol eines Gemisches aus Friedel-Crafts-Katalysator und Alkylaluminiumhalogenid verwendet werden. Werden Ausgangsstoffe mit Substituenten, die mit dem Katalysator stabile Komplexe bilden, verwendet, so ist die Gesamtkatalysatormenge entsprechend zu erhöhen. Die wirksame Menge der aluminiumorganischen Verbindung liegt vorteilhaft bei 0,1 bis ca. 1 mol Alkylaluminiumdihalogenid oder etwa 0,05 bis 0,5 mol Dialkylaluminiumhalogenid oder etwa 0,03 bis 0,33 mol Trialkylaluminium, jeweils bezogen auf ein Äquivalent Carbonsäurederivat wie Säurehalogenid bzw. -anhydrid. Größere Mengen bis hin zum völligen Ersatz des Aluminiumhalogenids sind möglich, jedoch wird man aus wirtschaftlichen Erwägungen versuchen, den Anteil der aluminiumorganischen Verbindung möglichst gering zu halten.

Die Reaktion kann ohne oder vorteilhaft in Gegenwart eines Lösungsmittels ausgeführt werden, wobei als Lösungsmittel die konventionellen Lösungsmittel für Friedel-Crafts-Reaktionen in Betracht kommen, z.B. Chlorbenzol, Dichlorbenzol, 1,2-Dichlorethan, Schwefelkohlenstoff, Nitromethan oder Nitrobenzol. Die Lösungsmittelmenge ist nicht kritisch, im allgemeinen können 100 bis 1000 g Lösungsmittel je Mol Alkylbenzol verwendet werden.

Die Durchführung der Reaktion kann in an sich bekannter Weise erfolgen, wobei man die Ausgangsstoffe bei Temperaturen von -20 bis 100, vorzugsweise 0 bis 60, insbesondere 10 bis 40°C und bei erhöhtem oder vermindertem Druck, vorzugsweise bei Normaldruck umsetzen kann.

Zweckmäßigerweise legt man das Carbonsäurehalogenid bzw. -anhydrid zusammen mit dem Lösungsmittel vor und gibt das Aluminiumhalogenid sowie anschließend den Alkylaromaten im Gemisch mit dem Metallalkyl oder Metallalkylhalogenid hinzu.

Die Aufarbeitung des Reaktionsgemisches und Isolierung der Produkte erfolgt in üblicher Weise, z.B. indem man das Reaktionsgemisch auf Wasser und/oder Eis gießt und nach Abtrennung der wäßrigen Phase das Keton durch Destillation oder Kristallisation gewinnt.

Nach dem erfindungsgemäßen Verfahren ist es überraschend möglich, die aromatischen Ketone, die wertvolle Zwischen- und Endprodukte beispielsweise für Farbstoffe, Hilfsstoffe, Pflanzenschutzmittel und Pharmaka sind, in gegenüber dem Stand der Technik höheren Ausbeuten herzustellen. Weiterhin können alkylsubstituierte, insbesondere tert.-alkylsubstituierte Aromaten acyliert werden, ohne daß eine starke Isomerisierung des Alkylrestes auftritt, was insbesondere im Fall der Synthese von (tert.-Amylbenzoyl)benzoesäure von Interesse ist, da diese ein wichtiges Zwischenprodukt zur Herstellung von tert.-Amylanthrachinon darstellt, das für die Produktion von Wasserstoffperoxid (s. DE-OS 20 13 299) benötigt wird.

<u>Beispiel 1</u>

Herstellung von 4-tert.-Butylacetophenon

78 g (1 mol) Acetylchlorid wurden in 100 ml Dichlorbenzol vorgelegt und dann 73 g (0,6 mol) $AlCl_3$ in Portionen hinzugegeben. Im Verlaufe von 5 Stunden wurde in diese Lösung bei 15 bis 20°C ein Gemisch von 134 g (1 mol) tert. Butylbenzol und 60 g (0,5 mol) Diethylaluminiumchlorid in 50 ml 1,2-Dichlorbenzol getropft. Anschließend wurde 1 Stunde bei 30°C nachgerührt.

Nach beendeter Reaktion wurde der Reaktionsaustrag auf ein Gemisch von 1 l $H_2O$ mit 300 g Eis und 30 ml konzentrierte $H_2SO_4$ gegossen, die organische Phase abgetrennt, getrocknet und destilliert.
Ausbeute: 162 g 4-tert.-Butylacetophenon (= 92 % d. Theorie).

<u>Beispiel 2</u>

Herstellung von 4-tert.-Amylpropiophenon

Entsprechend Beispiel 1 wurden umgesetzt:

92 g (1 mol) Propionsäurechlorid
148 g (1 mol) tert.-Amylbenzol
27 g (0,2 mol) $AlCl_3$
113 g (0,9 mol) Ethylaluminiumdichlorid

Ausbeute: 184 g 4-tert.-Amylpropiophenon (= 90 % d.Theorie).

<u>Beispiel 3</u>

Herstellung von 2-(4'-tert.-Amyl-benzoyl)-benzoesäure

Entsprechend Beispiel 1 wurden in 300 ml Dichlorbenzol umgesetzt:

74 g (0,5 mol) Phthalsäureanhydrid
74 g (0,5 mol) tert.-Amylbenzoyl
73 g (0,6 mol) $AlCl_3$
63 g (0,5 mol) Ethylaluminiumdichlorid

Ausbeute: 133 g 2-(4'-tert.-Amyl-benzoyl)-benzoesäure (=90 % d. Theorie).

<u>Vergleichsbeispiel 3a und 3b</u>

Die Umsetzung erfolgte entsprechend Beispiel 3 jedoch in Gegenwart von

a) 145 g (1,1 Mol) $AlCl_3$ ohne Zusatz von Ethylaluminiumdichlorid in 150 ml Dichlorbenzol.
b) Entsprechend Beispiel a) jedoch unter Einleiten von trockener Luft während der Umsetzung.

Nach beendeter Reaktion wurde der Reaktionsaustrag auf ein Gemisch von 1 l Wasser mit 300 g Eis und 30 ml konzentrierte $H_2SO_4$ gegossen, die organische Phase mit verdünnter Natronlauge extrahiert und die Amylbenzoyl-benzoesäuren aus der wäßrigen Phase mit Schwefelsäure ausgefällt und getrocknet. Man erhielt a) 120,5 g und b) 115 g eines Feststoffes, der gemäß HPLC-Analyse folgende Zusammensetzung aufwies (HPLC-Flächenprozente):

| | a) | b) |
|---|---|---|
| 2-(4-tert.-Amyl-benzoyl)-benzoesäure | 52% | 71% |
| 2-(4-sek.-Amyl-benzoyl)benzoesäure | 43% | 24% |
| Benzoylbenzoesäure | 2% | 2% |
| andere | 3% | 3% |

Beispiel 4

Herstellung von 2-(4'-tert.-Amylbenzoyl)-benzoesäure.

Entsprechend Beispiel 1 wurden in 300 ml Dichlorbenzol umgesetzt.

74 g (0,5 mol) Phthalsäureanhydrid
74 g (0,5 mol) tert.-Amylbenzol
81,3 g (0,6 mol) $AlCl_3$
51,4 g (0,25 mol) Methylaluminiumsesquichlorid $(CH_3)_3Al_2Cl_3$

Ausbeute: 139 g 2-(4'-tert.-Amylbenzoyl)-benzoesäure (= 94 % d. Theorie).

Beispiel 5

Herstellung von 2-[4'-(1-Ethyl-1-methyl-pentyl-benzoyl)]-benzoesäure

Entsprechend Beispiel 1 wurden umgesetzt:

74 g (0,5 mol) Phthalsäureanhydrid
95 g (0,5 mol) (1-Ethyl-1-methyl-pentyl)-benzol
113 g (0,85 mol) $AlCl_3$
30 g (0,25 mol) Diethylaluminiumchlorid

Ausbeute: 154 g 2-[4'-Ethyl-1-methyl-pentyl-benzoyl)]-benzoesäure (= 91 % d. Theorie).

Beispiel 6

Herstellung von 2-(4'-tert.-Amyl-benzoyl)-benzoesäure

Entsprechend Beispiel 3 wurden umgesetzt:

74 g (0,5 mol) Phthalsäureanhydrid
74 g (0,5 mol) tert.-Amylbenzol
97 g (0,8 mol) $AlCl_3$
34 g (0,3 mol) Triethylaluminium

Ausbeute: 127 g 2-(4'-tert.-Amylbenzoyl)-benzoesäure (= 86 % d. Theorie).

Beispiel 7

74 g (0,5 mol) Phthalsäureanhydrid wurden in 200 ml o-Dichlorbenzol vorgelegt und 128 g (1,05 mol) -$AlCl_3$ in Portionen bei 15 bis 20°C zugegeben. Im Verlaufe von 5 Stunden wurde bei dieser Temperatur ein Gemisch von 8,3 g (0,07 mol) Diethylzink und 74 g (0,5 mol) tert.-Amylbenzol zur Reaktionslösung getropft. Anschließend wurde 2 Stunden bei Raumtemperatur nachgerührt.

Nach beendeter Reaktion wurde der Reaktionsaustrag auf ein Gemisch von 1 l Wasser mit 300 g Eis und 30 ml konzentrierte $H_2SO_4$ gegossen, die organische Phase mit verdünnter Natronlauge extrahiert und die Amylbenzoyl-benzoesäuren aus der wäßrigen Phase mit Schwefelsäure ausgefällt und getrocknet. Man erhielt 133 g (90 % d. Theorie) eines Gemisches aus 2-(4'-tert.-Amyl-und 4'-sek.-Isoamylbenzoyl)-benzoesäure (Verhältnis tert./sek. = 79 : 21).

Beispiel 8

74 g (0,5 mol) Phthalsäureanhydrid wurden analog Beispiel 1 mit 128 g (1,05 mol) $AlCl_3$ und 25 g (0,2 mol) Diethylzink in 74 g tert.-Amylbenzol zur Reaktion gebracht. Nach der Aufarbeitung erhielt man 118 g (80 % d. Theorie) reine 2-(4'-tert.-Amylbenzoyl)-benzoesäure.

Beispiel 9

74 g (0,5 mol) Phthalsäureanhydrid und 134 g (1,10 mol) $AlCl_3$ wurden, wie unter Beispiel 1 beschrieben, mit 20 g (0,2 mol) Triethylbor in 74 g tert.-Amylbenzoyl in 8 Stunden zur Reaktion gebracht. Man erhielt nach der üblichen Aufarbeitung 127 g (86 % d. Theorie) eines Gemisches aus 2-(4'-tert.-Amyl- und 4'-sek.-Isoamylbenzoyl)-benzoesäure (Verhältnis tert./sek. = 60 : 40).

## Patentansprüche

1. Verfahren zur Acylierung von Aromaten nach Friedel-Crafts, dadurch gekennzeichnet, daß man die Acylierung in Gegenwart von Metallalkylen oder Metallalkylhalogeniden von Metallen oder Halbmetallen der zweiten bis fünften Hauptgruppe und/oder Metallen der zweiten oder vierten Nebengruppe des Periodensystems durchführt.
2. Verfahren nach Anspruch 1, dadurch gekennzeichnet, daß man Carbonsäurehalogenide oder Carbonsäureanhydride mit Alkylaromaten zu alkylsubstituierten aromatischen Ketonen umsetzt.
3. Verfahren nach den Ansprüchen 1 und 2, dadurch gekennzeichnet, daß man Metallalkyle oder Metallalkylhalogenide von Aluminium, Magnesium, Bor, Zinn, Zink oder Titan verwendet.
4. Verfahren nach den Ansprüchen 1 und 2, dadurch gekennzeichnet, daß man Trialkylaluminium oder Alkylaluminiumhalogenide der allgemeinen Formel I a
$R_nAlX_{3-n}$I a,
worin R gleiche oder verschiedene Alkylgruppen mit jeweils 1 bis 12 Kohlenstoffatomen darstellt, X Halogen bedeutet und n für die Zahlen 1, 2 oder 3 steht oder Gemische von zwei Verbindungen der Formel I verwendet.
5. Verfahren nach den Ansprüchen 1 bis 4, dadurch gekennzeichnet, daß man als Metallalkylhalogenide Metallalkylchloride oder -bromide verwendet.
6. Verfahren nach den Ansprüchen 1 bis 5, dadurch gekennzeichnet, daß man als Friedel-Crafts-Katalysator Aluminiumchlorid oder -bromid verwendet.
7. Verfahren nach den Ansprüchen 1 bis 6, dadurch gekennzeichnet, daß man als Aromaten Alkylbenzole verwendet.
8. Verfahren nach den Ansprüchen 2 bis 7, dadurch gekennzeichnet, daß man pro Äquivalent Säurehalogenid 1 bis 1,5 mol bzw. pro Äquivalent Säureanhydrid 2 bis 2,5 mol eines Gemisches des Friedel-Crafts-Katalysator und Trialkylaluminium oder Alkylaluminiumhalogenid verwendet.
9. Verfahren nach den Ansprüchen 2 bis 7, dadurch gekennzeichnet, daß man pro Äquivalent Säurehalogenid bzw. -anhydrid 0,1 bis ca. 1 mol Alkylaluminiumdihalogenid oder 0,03 bis ca. 0,33 mol Trialkylaluminium verwendet.

## Claims

1. A process for the acylation of an aromatic by the Friedel-Crafts method, wherein the acylation is carried out in the presence of a metalalkyl or metalalkyl halide of a metal or semimetal of main groups two to five and/or of a metal of subgroups two or four of the Periodic Table.
2. A process as claimed in claim 1, wherein a carbonyl halide or carboxylic anhydride is reacted with an alkylaromatic to give an alkyl-substituted aromatic ketone.
3. A process as claimed in claims 1 and 2, wherein a metalalkyl or metalalkyl halide of aluminum, magnesium, boron, tin, zinc or titanium is used.
4. A process as claimed in claims 1 and 2, wherein a trialkylaluminum or an alkylaluminum halide of the formule Ia
$R_nAlX_{3-n}$ Ia
where the radicals R are identical or different alkyl groups of 1 to 12 carbon atoms, X is halogen and n is 1, 2 or 3, or a mixture of two compounds of the formula I is used.
5. A process as claimed in claim 1 or 2 or 3 or 4, wherein the metalalkyl halide used is a metalalkyl chloride or bromide.
6. A process as claimed in claim 1 or 2 or 3 or 4 or 5, wherein the Friedel-Crafts catalyst used is aluminum chloride or bromide.
7. A process as claimed in claim 1 or 2 or 3 or 4 or 5 or 6, wherein the aromatic used is an alkylbenzene.
8. A process as claimed in claim 2 or 3 or 4 or 5 or 6 or 7, wherein a mixture of the Friedel-Crafts catalyst and the trialkylaluminum or alkylaluminum halide is used in an amount of from 1 to 1.5 moles per equivalent of acyl halide or in an amount of from 2 to 2.5 moles per equivalent of anhydride.
9. A process as claimed in claim 2 or 3 or 4 or 5 or 6 or 7, wherein from 0.1 to about 1 mole of alkylaluminum dihalide or from 0.03 to about 0.33 mole of trialkylaluminum is used per equivalent of acyl halide or anhydride.

## Revendications

1. Procédé d'acylation de composés aromatiques suivant Friedel-Crafts, caractérisé en ce qu'on effectue l'acylation en présence de métaux-alkyles ou d'halogénures de métaux-alkyles de métaux ou de métalloïdes du deuxième au cinquième groupe principal et/ou de métaux du deuxième ou du quatrième sous-groupe de la classification périodique des éléments.

2. Procédé suivant la revendication 1, caractérisé en ce que l'on fait réagir des halogénures d'acides carboxyliques ou des anhydrides d'acides carboxyliques avec des composés alkyl-aromatiques pour obtenir des cétones aromatiques substituées par des groupes alkyle.

3. Procédé suivant les revendications 1 et 2, caractérisé en ce que l'on utilise des métaux-alkyles ou des halogénures de métaux-alkyles d'aluminium, de magnésium, de bore, d'étain, de zinc ou de titane.

4. Procédé suivant les revendications 1 et 2, caractérisé en ce que l'on utilise un aluminium-trialkyle ou des halogénures d'aluminium-alkyle de la formule générale Ia

$R_nAlX_{3-n}$ Ia

dans laquelle R représente des groupes alkyle identiques ou différents renfermant chacun 1 à 12 atomes de carbone, X représente un halogène et n représente les nombres 1, 2 ou 3, ou bien des mélanges de deux composés de la formule I.

5. Procédé suivant les revendications 1 à 4, caractérisé en ce que l'on utilise, comme halogénures de métaux-alkyles, des chlorures ou des bromures de métaux-alkyles.

6. Procédé suivant les revendications 1 à 5, caractérisé en ce que l'on utilise, comme catalyseur de Friedel-Crafts, du chlorure ou du bromure d'aluminium.

7. Procédé suivant les revendications 1 à 6, caractérisé en ce que l'on utilise, comme composés aromatiques, des alkyl-benzènes.

8. Procédé suivant les revendications 2 à 7, caractérisé en ce que l'on utilise, par équivalent d'halogénure d'acide, 1 à 1,5 mole, ou, par équivalent d'anhydride d'acide, 2 à 2,5 moles d'un mélange du catalyseur de Friedel-Crafts et d'aluminium-trialkyle ou d'halogénure d'aluminium-alkyle.

9. Procédé suivant les revendications 2 à 7, caractérisé en ce que l'on utilise, par équivalent d'halogénure ou d'anhydride d'acide, 0,1 à environ 1 mole de dihalogénure d'aluminium-alkyle ou 0,03 à environ 0,33 mole d'aluminium-trialkyle.